# EUROPEAN PATENT APPLICATION

(11) **EP 4 011 431 A1**
(43) Date of publication of application: **15.06.2022**
(21) Application number: 20852968.5
(22) Date of filing: 07.08.2020
(51) Int. Cl.: A61M 25/098

(54) **CATHETER AND DIAGNOSTIC METHOD**

(30) Priority: 13.08.2019 JP 2019148492
(71) Applicant: TERUMO Kabushiki Kaisha, Tokyo 151-0072 (JP)
(72) Inventor: NISHIDE, Ayaka, Tokyo 163-1450 (JP); SAITO, Yoshiyuki, Tokyo 163-1450 (JP); KANAZAWA, Yuya, Fujinomiya-shi, Shizuoka 418-0015 (JP); HAMURO, Kouta, Fujinomiya-shi, Shizuoka 418-0015 (JP); FUKUOKA, Tetsuya, Ashigarakami-gun, Kanagawa 259-0151 (JP)
(74) Representative: Casalonga
(86) International application number: PCT/JP2020/030338
(87) International publication number: WO 2021/029348

(57) **Abstract**

Provided are: a catheter having an X-ray imageable catheter marker that enables fast identification of the in vivo orientation of a catheter tip; and a diagnostic method. An X-ray imageable catheter marker (100) is disposed asymmetrically with respect to the center of a shaft center line (A) of a catheter. X-ray images of different shapes under X-ray imaging are thus obtained, and the catheter position and tip orientation can be easily determined.

## Description

### Technical Field

The present invention relates to a catheter and a diagnostic method.

### Background Art

A catheter includes a hollow shaft including a distal end and a proximal end, and a hand-side unit such as a hub that is provided at the proximal end of the shaft. An operator disposes the distal end of the shaft to a position and an orientation as a target, while changing a position or an orientation of the distal end of the shaft by a forward, backward, or rotational operation of the hand-side unit if necessary inside the body of a patient under the radiopaque.

### Citation List

### Patent Literature

PTL 1: JP-A-7-8563
PTL 2: JP-A-2001-353225

### Summary of Invention

### Technical Problem

In catheters, a radiopaque marker is attached to a shaft in order to allow a position and an orientation of the catheter in a blood vessel to be observed through an angiogram.

Examples of the structure including the radiopaque marker can include a structure in which a tubular distal tip including metal powders having a radiopaque property such as tungsten and resin being mixed together is attached to a distal end of a shaft, and a structure in which a tubular marker including an iridium alloy is fixed to a balloon inner tube shaft including a guide wire lumen of a balloon catheter.

When the distal tip or the tubular marker each having a cylindrical shape is attached to a distal end or the like of a hollow shaft typically having a circular cross-section so as to be coaxial with the center of the shaft, the shaft and the tubular marker or the like become left-right symmetrical with a symmetrical plane passing through the center of the shaft. In other words, the tubular marker or the like and the shaft have a symmetrical shape that is left-right symmetrical with respect to a so-called longitudinal section.

Accordingly, even when a hand-side unit is turned about the center of the shaft as a rotation axis in the blood vessel, and the incident direction of X-rays is changed such that an X-ray source of an radiopaque device goes around a patient, an angiogram of the distal tip or the tubular marker having a simple cylindrical shape does not change, and no difference occurs.

For example, in a case where a portion of the shaft in the vicinity of the distal end is bent in a dogleg shape to be shaped, in a case where a plane made by the shaped portion of the distal end of the shaft and the X-ray incident direction are parallel to each other, it is difficult to determine whether an opening portion that is present at the distal end of the shaft of the catheter faces an orientation moving closer to or moving away from the operator for a short time in some cases.

### Solution to Problem

The invention below attains the above object.

(1) A catheter according to the invention is characterized in that: the catheter includes a hollow shaft including a distal end and a proximal end, a hand-side unit provided to the proximal end of the shaft, and a catheter marker having an radiopaque property; the shaft includes at least one symmetrical plane; the catheter marker includes a first marker and a second marker; the first marker and the second marker have different shapes, and are arranged to the shaft by being spaced from each other; and the catheter marker has a left-right asymmetrical shape with respect to the symmetrical plane.
(2) The catheter may be the catheter according to the abovementioned (1), in which the catheter marker includes at least one among a welding point of metal wires, an overlapping portion of metal wires that forms a reinforcement body, and a catheter opening portion that contains an radiopaque agent.
(3) The catheter may be the catheter according to the abovementioned (1), in which the catheter marker forms at least three angiograms having different shapes depending on an incident direction of X-rays.
(4) The catheter may be the catheter according to the abovementioned (1) in which at least one of the first marker and the second marker has a left-right asymmetrical shape with respect to the symmetrical plane.
(5) A diagnostic method according to the invention is a diagnostic method of a distal end orientation of a catheter inside a body of a patient, characterized in that the diagnostic method includes: a step of preparing the catheter that includes a hollow shaft including a distal end and a proximal end, a hand-side unit provided to the proximal end of the shaft, and a catheter marker having an radiopaque property, the shaft includes at least one symmetrical plane, the catheter marker includes a first marker and a second marker, the first marker and the second marker have different shapes, and are arranged to the shaft by being spaced from each other, and the catheter marker has a left-right asymmetrical shape with respect to the symmetrical plane; and a determination step of determining the distal end orientation of the catheter from angiograms having different shapes depending on an incident direction of X-rays.

### Advantageous Effect of Invention

The catheter according to the invention, the shape of the catheter marker is left-right asymmetrical with respect to the shaft including a symmetrical plane that is left-right symmetrical, so that angiograms having different shapes depending on the incident direction of X-rays are formed.

Accordingly, when the hand-side unit is turned using the center of the shaft as the rotation axis to rotate the shaft, the shape of the angiogram of the catheter marker changes. Therefore, the operator can identify for a short time an orientation of the distal end portion of the shaft in the blood vessel.

Moreover, the catheter attains easy recognition of the distal end orientation of the catheter and easy selection determination in a tortuous portion or a bifurcated portion, so that the catheter can easily reach a lesion area along a guide wire to shorten the surgery time, thereby allowing the reduction in the burden of a patient, and the reduction in labor costs.

### Brief Description of Drawings

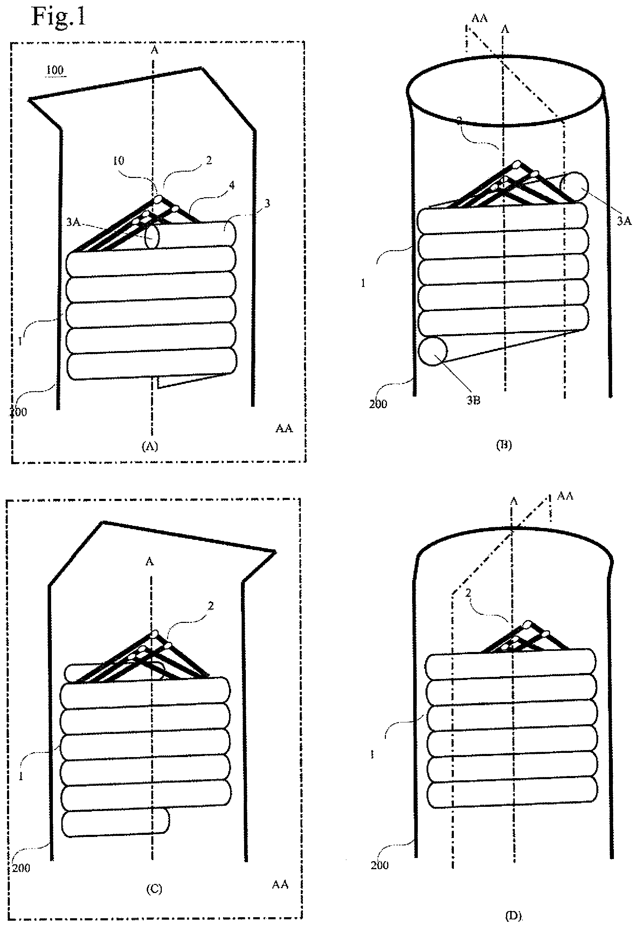

[Figs. 1(A) to 1(D)] Figs. 1(A) to 1(D) are planar perspective views in which a distal end portion of a shaft in a first embodiment according to the invention is stepwisely rotated in a clockwise direction viewed from a proximal end: (A) illustrates a state at 0°; (B) illustrates a state where the distal end portion of the shaft is rotated by 90°, (C) illustrates a state rotated by 180°; and (D) illustrates a state rotated by 270°.

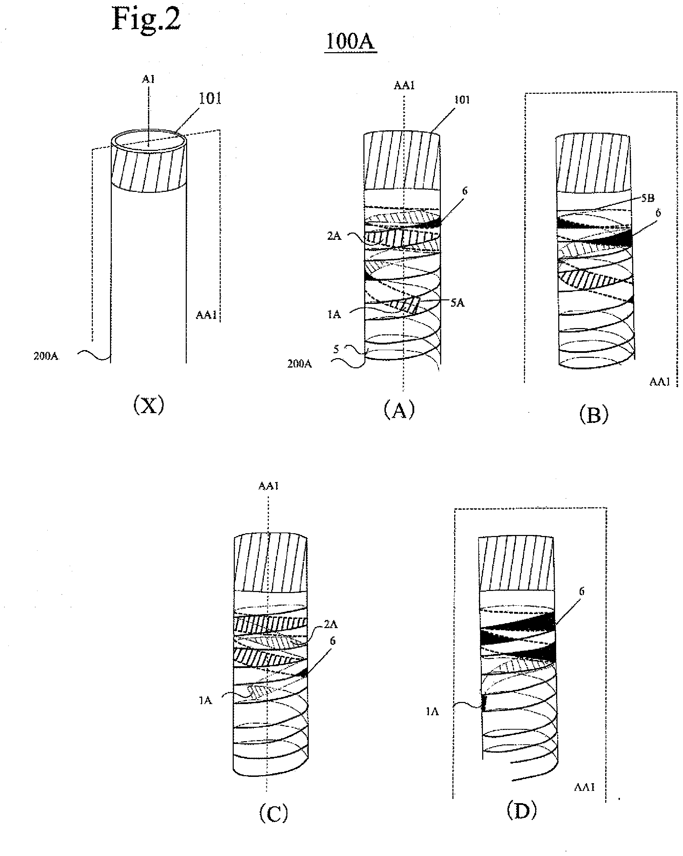

[Figs. 2(X) to 2(D)] Figs. 2(X) to 2(D) are views illustrating a distal end portion of a shaft in a second embodiment according to the invention: (X) is a perspective view, (A) is a planar perspective view illustrating a state at 0°; (B) is a planar perspective view illustrating a state where the distal end portion of the shaft is rotated by 90° in a clockwise direction viewed from a proximal side; (C) is a planar perspective view illustrating a state rotated by 180°; and (D) is a planar perspective view illustrating a state rotated by 270°.

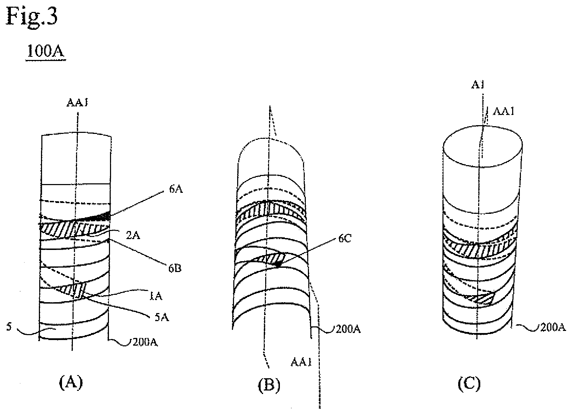

[Figs. 3(A) to 3(C)] Figs. 3(A) to 3(C) are views illustrating the distal end portion of the shaft: (A) is a planar perspective view of the distal end portion of the shaft; (B) is a planar perspective view of the shaft viewed from obliquely above at the proximal side; and (C) is a planar perspective view of the shaft viewed from obliquely above at a distal side.

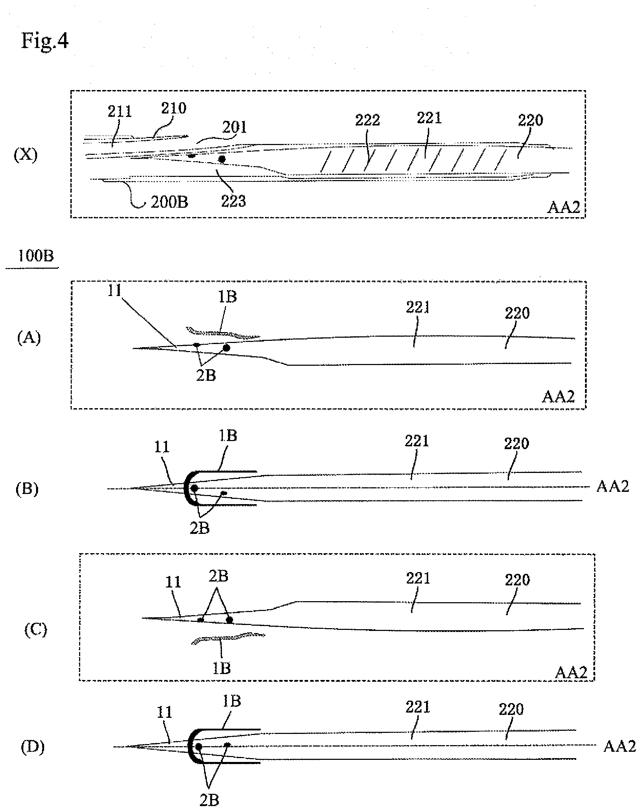

[Figs. 4(X) to 4(D)] Figs. 4(X) to 4(D) are views illustrating a rapid exchange type (RX) catheter in a third embodiment according to the invention: (X) is a vertical cross-sectional view in the vicinity of a symmetrical plane of a proximal side guide wire opening portion of the shaft: (A) is a planar perspective view under the radiopaque; (B) is a planar perspective view illustrating a state where the shaft is rotated by 90° in a clockwise direction viewed from a proximal end; (C) is a planar perspective view illustrating a state rotated by 180°; and (D) is a planar perspective view illustrating a state rotated by 270°.

### Description of Embodiments

Hereinafter, preferred embodiments of the invention are provided, and are described in details with reference to the accompanying drawings. Note that, the size ratios in the drawings may be exaggerated for convenience of explanation, and may be different from the actual ratios in some cases.

Note that, a symmetrical plane in which a surface shape of a shaft becomes left-right symmetrical (plane symmetrical) corresponds any longitudinal section that passes through the center line serving as a long axis in a case of a catheter having a simple cylindrical shape, or a longitudinal section in which the surface shape of the shaft is left-right symmetrical, in a case of a complicated structure including two lumens.

In the case of the cylindrical shape, the number of the symmetrical planes passing through the center of the shaft becomes infinite, and the same applies to the distal end taper. Alternatively, a shaft in which a distal end opening portion is obliquely cut, or a shaft having a double lumen structure of including both of a guide wire lumen and an inflation lumen includes at least one symmetrical plane.

A shaft 200 of a catheter according to a first embodiment illustrated in Figs. 1(A) to 1(D) includes an outer layer and an inner layer (not illustrated), and forms therebetween a braid 4 in which metal wires are woven together as a reinforcement body.

An radiopaque metal wire 3, which is a different metal wire, for example, gold, is coil-wound around an outer side of the braid 4 to form a coil marker, which serves as a first marker 1. Further, the braid 4 is cut out, and uses a welding portion 10 generated when an intersection portion of the braids 4 is welded, as a second marker 2.

Materials for the braid 4 may be, for example, tungsten or a stainless steel wire, and the thickness of the braid 4 is not specifically limited, the braid 4 having a diameter of 5 to 100 µm, preferably 15 to 60 µm, is preferable. The cross-section of the braid 4 is circular, and may be elliptical, rectangular, or oval.

Moreover, as the woven structure of metal wires, a single wire as one of the metal wires and two multiple wires as the other may be crossed and woven together, or single wires or multiple wires may be crossed and woven together. Moreover, one and the other of the metal wires may have different thicknesses or different types, respectively.

The first marker 1 is a coil marker, includes a distal side cut plane 3A and a proximal side cut plane 3B, and includes a center line A, which is a long axis of the shaft 200, and the shape of the coil marker is left-right asymmetrical with respect to a symmetrical plane AA to which a surface shape of the shaft 200 is left-right symmetrical.

An angiogram of the three-dimensional coil marker is projected on a plane of an X-ray detector, the shape of which is left-right asymmetrical with respect to the center A of the shaft 200.

A distance from a distal end of the welding portion 10 to a distal end of the distal side cut plane 3A may be determined as appropriate, and exceeds 0 and equal to or less than 1 mm, more preferably 0.1 to 0.5 mm when the outer diameter of the catheter is equal to or less than 1 mm.

In a catheter marker 100, the first marker 1 and the second marker 2 are combined to cause the catheter marker 100 have a left-right asymmetrical shape with respect to the symmetrical plane AA.

An angiogram of the three-dimensional catheter marker 100 is projected on the plane of the X-ray detector, similarly, as illustrated in Figs. 1(B) and (D), the shape of which is left-right asymmetrical with respect to the symmetrical plane AA.

When the shaft 200 is rotated using the center line A of the shaft 200 as a rotation axis while turning the hand-side unit, the shape of the catheter marker 100 is seen by being changed viewed from an observer who observes the angiogram. Similarly, the shape of the angiogram of the catheter marker 100 is changed by the rotation of the shaft 200.

Accordingly, an orientation of a distal end portion of the shaft 200 can be identified with the change in the shape of the angiogram of the catheter marker 100.

With the catheter marker 100 formed by the first marker 1 and the second marker 2 in this manner, as illustrated in Fig. 1(A), Fig. 1(B) in which the shaft 200 is rotated by 90° in a clockwise direction viewed from the proximal side, Fig. 1(C) rotated by 180°, and Fig. 1(D) rotated by 270°, the shapes of the angiograms of the catheter marker 100 are different from one another.

Specially, in a case where a plane made by the shape of the distal end portion of the shaft 200 and a base portion of the shaft 200 is parallel to an incident direction of X-rays, specifically in Fig. 1(B) and Fig. 1(D), the distal end orientation of the shaft 200 is difficult to be identified for a short time only from the angiograms of the shaft 200.

However, when the shapes of the first coil marker 1 are compared, both of the distal end 3A and the proximal end 3B of the gold wire 3 can be visually recognized in Fig. 1(B), whereas neither the distal end 3A nor the proximal end 3B is hidden in the gold wire 3, and thus can be visually recognized in Fig. 1(D).

Accordingly, it is possible to identify for a short time whether a distal end orientation of a distal end of the shaft 200, specifically, of a distal end opening portion of the catheter is an orientation in Fig. 1(B) moving closer to or an orientation in Fig. 1(D) moving away from the operator.

In addition, when Fig. 1(A) and Fig. 1(C) in which the shaft 200 is rotated by 180° are compared with each other, positions and relative sizes of the distal end and the proximal end of the first marker 1 with respect to the second marker 2 are different from each other.

In this manner, with the catheter marker 100 having a left-right asymmetrical shape with respect to the symmetrical plane AA, the incident direction of X-rays is changed, for example, the hand-side unit is turned using the center line A of the shaft 200 as the rotation axis, at least four angiograms having different shapes can be obtained on the basis of the shapes of the catheter marker 100.s

Accordingly, the orientation of the distal end portion of the shaft 200 can be identified for a short time due to the difference in the shape of the angiograms of the catheter marker 100.

Alternatively, the motion of the hand-side unit and an angiogram are recorded, and the orientation of the distal end portion of the shaft 200 may be determined using the data with the image recognition by artificial intelligence, which is preferable in particular for a case of a thin catheter, such as a micro catheter, the diameter of which is equal to or less than 1 mm, and the orientation of which is difficult to be determined by the naked eye.

In a second embodiment, a shaft 200A includes AA1 as a symmetrical plane, as illustrated in Fig. 2(X).

The shaft 200A includes an inner layer and an outer layer, and a reinforcement body including a coil-wound coil wire 5 that is disposed between the inner layer and the outer layer, and when the coil wire 5 is wound in an overlapped manner, the coil wire 5 becomes left-right asymmetrical with respect to the symmetrical plane AA1 of the shaft.

The cross-sectional shape of the coil wire 5 may be circular, elliptical, rectangular, or oval, and the material thereof may be a stainless steel wire and the like. When the cross-sectional shape of the coil wire 5 is rectangular, the width is 10 to 500 µm, preferably 100 to 300 µm, and the thickness is 5 to 200 µm, preferably 15 to 100 µm.

The manufacturer forms the coil wire 5 by coil-winding a plate metal wire made of stainless steel as a reinforcement body on the outer side of inner layer resin (not illustrated) coated on a mandrel (not illustrated).

The coil wire 5 is wound around the outer side of the inner layer resin from a coil wire end portion 5A toward a distal side of the shaft 200A. After the coil wire 5 has been wound to a prescribed position that serves as a coil wire distal end 5B, the coil wire 5 is wound while being overlapped continuously to the outer side of the wound coil wire 5 (outer side in a radial direction of the shaft 200A) toward the proximal side.

The coil wire end portion 5A is pressed down by the coil wire 5 wound on the outer side in an overlapped manner.

After the coil wire 5 has been wound to the shaft proximal end, the outer side of the reinforcement body formed by the coil wire 5 is coated with outer layer resin (not illustrated), so that the coil wire 5 is fixed by being sandwiched between the resin of the inner layer and the resin of the outer layer, and is prevented from spreading out. The method of winding the coil wire 5 may be determined as appropriate, regardless of this order.

The coil wire end portion 5A may be at a further proximal side than the coil wire distal end 5B, and the position from the coil wire distal end 5B to the coil wire end portion 5A is preferably equal to or less than 10 winding of the coil wire, more preferably equal to or less than 5 winding, which results in easy manufacturing.

When the outer diameter of the catheter is 2 to 3 mm, a distance from the coil wire distal end 5B to the coil wire end portion 5A is preferably 1 to 10 mm, more preferably 3 to 6 mm.

Moreover, as illustrated in Fig. 2(A), the two coil wires 5 are overlapped to increase the radiopaque property, and can be used as a marker.

A catheter marker 100A formed by overlapping the coil wires 5 with each other includes a first marker 1A formed by overlapping the coil wire end portion 5A at the most proximal side and the coil wires 5 with each other, and a second marker 2A in which the coil wires 5 are overlapped with each other at the further distal side than the first marker 1A.

As illustrated in Fig. 2(A), the length and the angle when the coil wires cross each other are different between the first marker 1A and the second marker 2A, so that the first marker 1A and the second marker 2A have different shapes.

In Figs. 2(X) to 2(D), five portions in which the coil wires 5 are brought direct contact and overlapped with each other (portions in which the coil wires are overlapped with each other in the radial direction of the shaft 200A) are formed, so that another overlapping portion may be used as the second marker 2A, or all the overlapping portions other than the first marker 1A may be used as the second markers 2A.

The first marker 1A and the second marker 2A become left-right asymmetrical with respect to the symmetrical plane AA1 having a shaft center line A1.

In addition, the angiogram varies depending on the incident direction of X-rays to the shaft 200A.

Specially, portions illustrated by black coating, in each of the coil wire wound in the inner side and the coil wire wound in the outer side, a hand-side marker in Figs. 2(X) to 2(D) and a back-side marker are overlapped with each other in the drawings, are seen that the four coil wires 5 are overlapped. Therefore, on the angiogram, a high radiopaque property region 6 having a higher radiopaque property is formed.

In Fig. 2(A), the high radiopaque property regions 6 are disposed on both ends of the shaft 200A. In Fig. 2(B) in which the shaft 200A is rotated by 90° in a clockwise direction viewed from the proximal side, the high radiopaque property regions 6 are seen with the larger area. In Fig. 2(C) in which the shaft 200A is further rotated by 90°, in other words, rotated by 180° with respect to the state in Fig. 2(A), on the other hand, the area of the high radiopaque property regions 6 becomes smaller. In Fig. 2(D) in which the shaft 200A is further rotated by 90°, rotated by 270° with respect to the state in Fig. 2(A), the area and the number of the high radiopaque property regions 6 are increased.

angiograms with the catheter marker 100A in Figs. 2(A) to (D) have different shapes, respectively. When a hand-side unit of the catheter marker 100A is turned or the X-ray source is turned, the incident direction of X-rays with respect to the shaft 200A changes, so that at least four angiograms having different shapes of the catheter marker 100A can be obtained.

As describe above, the catheter marker 100A has a left-right asymmetrical shape with respect to the symmetrical plane AA1 to which the shaft 200A is left-right symmetrical. Therefore, by turning the hand-side unit of the shaft 200A, at least four angiograms having different shapes can be obtained on the basis of the shapes of the catheter marker 100A.

Therefore, the operator can identify the distal end orientation of the shaft 200A for a short time due to the difference in the shapes of the angiograms of the catheter marker 100A.

Alternatively, in order to identify a distal end position of the shaft 200A, a resin chip having a high radiopaque property on the shaft distal end may be used as a different marker 101.

Moreover, as in Figs. 3(A) to 3(C), with respect to Fig. 3(A) in which the coil wire end portion 5A is viewed from directly above (direction perpendicular to the shaft center line A1), in Fig. 3(B) in which the coil wire end portion 5A is viewed from obliquely above at the shaft base portion side and in Fig. 3(C) viewed from obliquely above at the shaft distal side, an overlap between the hand-side coil wire and the back-side coil wire (not illustrated) of the shaft 200A changes.

Specifically, in Fig. 3(A), high radiopaque regions 6A and 6B formed by overlapping four middle coil wires 5 are present, whereas, in Fig. 3(B), the high radiopaque regions 6A and 6B disappear, and a high radiopaque region 6C is newly formed. Meanwhile, in Fig. 3(C), the high radiopaque region 6 disappears.

In this case, not only the shape but also the shade of the coil wire 5 in the angiogram changes. Depending on the relative position and/or size of the first marker 1A and the second marker 2B, on the basis of the shapes of the catheter marker 100A, different angiograms having at least three shapes can be obtained.

Accordingly, the operator can identify for a short time, due to the change in the shape of the angiograms of the catheter marker 100A, whether the distal end portion of the shaft faces an orientation moving closer to or moving away from the operator.

As a third embodiment, a catheter in which a catheter marker is arranged to a rapid exchange (RX) type catheter will be described.

Fig. 4(X) is a cross-sectional view by a symmetrical plane AA2 of a proximal-side guide wire opening portion 201 (catheter opening portion) of a guide wire lumen 211 of the RX catheter, which is used in a balloon catheter or a stent delivery catheter, for example.

A shaft 200B includes the guide wire lumen 211 and an inflation lumen 223, and has a left-right symmetrical shape with respect to the symmetrical plane AA2.

The shaft 200B includes a distal end guide wire shaft 210 at the distal side that forms the guide wire lumen 211. Resin containing radiopaque powders is disposed to the proximal-side guide wire opening portion 201 of the distal end guide wire shaft 210 of the shaft 200B to form a first marker 1B. In addition, in the inflation lumen 223 that extends from a proximal side of the shaft 200B, a proximal portion 220 that includes a metal pipe 221 with slits 222 being provided is formed. A distal end of the metal pipe 221 includes a reduced diameter portion 11 in which the diameter is reduced.

The second marker 2B such as a disc-shaped iridium alloy having an radiopaque property is provided to the reduced diameter portion 11, whereby it is possible to easily determine an orientation of the proximal-side guide wire opening portion 201 of the guide wire shaft 210. A plurality of second markers 2B, the respective shapes and lengths of which are varied, may preferably be provided, alternatively, may have the same shape such as an elliptical or oval shape and be provided to different positions and at different angles with respect to the symmetrical plane AA2.

When the outer diameter of the catheter is from 1 to equal to or less than 2 mm, a shortest distance between the first marker 1B and the second marker 2B closest to the first marker 1B is equal to or more than 0 and equal to or less than 10 mm, more preferably 0. 1 to 5 mm in the long axis direction, as illustrated in Fig. 4(B), and in a top view Fig. 4(C), equal to or more than 0 and equal to or less than 1.5 mm, more preferably 0.1 to 1 mm, in a vertical direction with respect to the long axis.

In the third embodiment, two elliptical-shaped markers having the same shape are arranged as the second markers 2B at positions left-right asymmetrical with respect to the symmetrical plane AA2.

The first marker 1B and the second marker 2B in the proximal-side guide wire opening portion 201 of the shaft 210 have different shapes, and are arranged so as to be spaced from each other to form a catheter marker 100B.

Accordingly, when an operator turns a hand-side unit of the RX catheter to rotate the shaft, as illustrated in Figs. 4(A) to (D), the operator can obtain at least four angiograms having different shapes of the catheter marker 100B, and can determine an orientation of the proximal-side guide wire opening portion 201 of the shaft, and a distal end orientation of the corresponding shaft 200B.

Therefore, the operator can grasp the orientation of the catheter and cause the catheter to follow the guide wire and pass through a bifurcated blood vessel of the coronary artery, a tortuous blood vessel of the hepatic artery, or a branched blood vessel of the lower limb artery.

In the above, the present invention has been described with the preferred embodiments, however, the present invention is not limited to the embodiments, and it is needless to say that various modifications are possible without deviating from the scope of the invention.

For example, in the second embodiment, the orientation of the catheter may be determined due to the change in the shape of the portion where metal wires are not overlapped with each other or the gap where no metal wire is present, not the marker portion that is formed by metal wires having an radiopaque property. The arrangement of the markers so as to be spaced from each other may include the arrangement as in Fig. 3 in which the markers are spaced by a distance that allows the shapes thereof to change depending on the direction from which the markers are seen, or may be the arrangement as in Figs. 1(A) to 1(D) in which the markers are arranged so as to be close to each other. Alternatively, the arrangement in which the marker are seen as being spaced from each other under the radiopaque, the arrangement in which the marker are seen as being overlapped with each other in the irradiation direction of the X-rays may be employed.

Note that, the application is based upon and claims the benefit of priority of the prior Japanese Patent Application No. 2019-148492, filed on August 13, 2019, the entire contents of which are incorporated herein by reference.

### Reference Signs List

1, 1A, 1B first marker
2, 2A, 2B second marker
3 radiopaque metal wire
3A distal side cut plane
3B proximal side cut plane
4 braid
5 coil wire
5A coil wire end portion
5B coil wire distal end portion
6 high radiopaque property region
10 welding portion
11 reduced diameter portion
100, 100A, 100B catheter marker
101 marker
200, 200A, 200B shaft
201 proximal side guide wire opening portion (catheter opening portion)
A, A1 shaft center line
AA, AA1, AA2 shaft symmetrical plane

## Claims

1. A catheter, **characterized in that**:
the catheter comprises a hollow shaft including a distal end and a proximal end, a hand-side unit provided to the proximal end of the shaft, and a catheter marker having a radiopaque property;
the shaft includes at least one symmetrical plane;
the catheter marker includes a first marker and a second marker;
the first marker and the second marker have different shapes, and are arranged to the shaft by being spaced from each other; and
the catheter marker has a left-right asymmetrical shape with respect to the symmetrical plane.

2. The catheter according to claim 1, wherein the catheter marker includes at least one among a welding point of metal wires, an overlapping portion of metal wires that forms a reinforcement body, and a catheter opening portion that contains a radiopaque agent.

3. The catheter according to claim 1 or 2, wherein the catheter marker forms at least three angiograms having different shapes depending on an incident direction of X-rays.

4. The catheter according to any one of claims 1 to 3, wherein at least one of the first marker and the second marker has a left-right asymmetrical shape with respect to the symmetrical plane.

5. A diagnostic method of a distal end orientation of a catheter inside a body of a patient, **characterized in that** the diagnostic method comprises:
a step of preparing the catheter that includes a hollow shaft including a distal end and a proximal end, a hand-side unit provided to the proximal end of the shaft, and a catheter marker having a radiopaque property, the shaft including at least one symmetrical plane, the catheter marker including a first marker and a second marker, the first marker and the second marker having different shapes, and being arranged to the shaft by being spaced from each other, and the catheter marker having a left-right asymmetrical shape with respect to the symmetrical plane; and
a determination step of determining the distal end orientation of the catheter from angiograms having different shapes depending on an incident direction of X-rays.
